(19) **Europäisches Patentamt / European Patent Office / Office européen des brevets**

(11) **EP 4 560 005 A1**

(12) **EUROPEAN PATENT APPLICATION**
published in accordance with Art. 153(4) EPC

(43) Date of publication:
28.05.2025 Bulletin 2025/22

(21) Application number: 23903698.1

(22) Date of filing: 27.09.2023

(51) International Patent Classification (IPC):
$C12N\ 1/12^{(2006.01)}$    $C12P\ 7/6434^{(2022.01)}$
$A23K\ 10/16^{(2016.01)}$    $A23K\ 20/158^{(2016.01)}$
$A23L\ 29/00^{(2016.01)}$    $A23L\ 33/12^{(2016.01)}$
$C12R\ 1/89^{(2006.01)}$

(52) Cooperative Patent Classification (CPC):
A23K 10/12; A23K 10/16; A23K 20/158;
A23L 17/60; A23L 29/00; A23L 33/12; C12N 1/12;
C12P 7/6434; C12R 2001/89

(86) International application number:
PCT/KR2023/015037

(87) International publication number:
WO 2024/128494 (20.06.2024 Gazette 2024/25)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 15.12.2022 KR 20220175630

(71) Applicant: CJ Cheiljedang Corporation
Seoul 04560 (KR)

(72) Inventors:
• JANG, Sunghoon
  Seoul 04560 (KR)
• CHOI, Jung-Woon
  Seoul 04560 (KR)
• SHIN, Won Sub
  Seoul 04560 (KR)
• KANG, Hae-Won
  Seoul 04560 (KR)
• RYU, Ae Jin
  Seoul 04560 (KR)
• GWAK, Jun Seok
  Seoul 04560 (KR)
• KIM, Ji Young
  Seoul 04560 (KR)
• JEON, Seungjib
  Seoul 04560 (KR)

(74) Representative: Meissner Bolte Partnerschaft
mbB
Patentanwälte Rechtsanwälte
Postfach 86 06 24
81633 München (DE)

Remarks:
The complete document including Reference
Table(s) and the Sequence Listing(s) can be
downloaded from the EPO website

(54) **NOVEL SCHIZOCHYTRIUM SP. STRAIN AND METHOD FOR PRODUCING OIL CONTAINING OMEGA-3 USING SAME**

(57)    A Schizochytrium sp. strain, which is a high-bio-oil-producing microalgae containing a high concentration of DHA, of the present invention, has high bio-oil productivity due to a high crude fat content in a produced biomass, and has the characteristic of containing a high content of docosahexaenoic acid within cells. Accordingly, the strain itself, a biomass produced by culturing and fermenting the strain, and its concentrates and extracts can be helpfully used as a feed composition or food composition.

**(Cont. next page)**

EP 4 560 005 A1

【FIG. 1】

**Description**

[TECHNICAL FIELD]

Cross-reference to related application(s)

The present application claims the benefit of the priority based on Korean Patent Application No. 10-2022-0175630 filed on December 15, 2022, and the entire contents disclosed in the documents of the corresponding Korean patent application are incorporated as a part of the present description.

The present application relates to a novel *Schizochytrium* sp. strain containing high intracellular docosahexaenoic acid and a method for producing oil containing omega-3 using the same.

[BACKGROUND ART]

Thraustochytrid survives and is distributed in various environments in the natural world. It attaches to an organism and live in symbiosis, or floats in marine environments, or freshwater, or brackish water environments, and is distributed and survives in various sedimentary topography layers. Such a thraustochytrid belongs to the lowest level of the marine ecological food chain, and is also classified as an organic heterotrophic protist microalgae as phytoplankton. Thraustochytrid in the natural environment functionally plays a role of circulation and purification of natural circulation elements such as sulfur, nitrogen, phosphorus, potassium and the like. **In** addition, it functions as a source in a marine ecosystem by containing a high concentration of polyunsaturated fatty acids (PUFA) including docosahexaenoic acid (DHA) and eicosapentaenoic acid (EPA) classified to omega-3.

Most higher organisms including humans cannot synthesize polyunsaturated fatty acids including docosahexaenoic acid and eicosapentaenoic acid by themselves, so they should be consumed as essential nutrients. Docosahexaenoic acid and eicosapentaenoic acid among polyunsaturated fatty acids are essential fatty acids for the brain, eye tissue, and nervous system, and in particular, they are known to play an important function in development of the nervous system such as vision and motor nerve ability of infants and the like and prevention of cardiovascular disease, and they are the most abundant components in structural lipids of the brain.

Until now, the main source of polyunsaturated fatty acids is fish oil extracted from oil of blue fish such as mackerel, saury, tuna, horse mackerel, sardine, herring and the like, which is also very useful as fish farming feed such as seawater fish initial feed. Extraction and intake of polyunsaturated fatty acids from fish oil has been industrially developed, but there are also disadvantages. The quality of fish oil varies depending on the fish species, season and fishing location, and it is generated through fishing, so there are difficulties in continuous supply. In addition, there are limitations in the manufacturing process and production due to contamination problems by heavy metals and organic chemicals comprised in fish oil, problems of oxidizing double bonds during the processing process as well as unique fishy smell of fish oil, and the like.

In order to solve such problems, research has recently been conducted on methods for production of polyunsaturated fatty acids including docosahexaenoic acid and eicosapentaenoic acid by microbial culture. In particular, microalgae can provide various advantages compared to fish oil, in addition to the ability to newly synthesize fatty acids naturally. It can be stably supplied through industrial-scale culture and enables production of biomass with a relatively constant biochemical composition. Unlike fish oil, lipids produced by microalgae do not have any unpleasant odor. Furthermore, it has a simpler composition than fish oil, and this makes it easier to separate major fatty acids.

Based on these advantages, research on production of polyunsaturated fatty acids including omega-3 un-saturated fatty acids ($\omega$-3 unsaturated fatty acids) such as docosahexaenoic acid (DHA), eicosapentaenoic acid (EPA), arachidonic acid (ARA), docosapentaenoic acid (DPA), and $\alpha$-linolenic acid, and the like using microalgae has been recently progressed very rapidly, and it is mainly production of polyunsaturated fatty acids by *Thraustochytrium* sp. and *Schizochytrium* sp. microorganisms, which are types of marine microalgae. For example, a method for producing omega-3 polyunsaturated fatty acids using *Schizochytrium* sp. ATCC20888 and *Schizochytrium* sp. PTA10208 which are *Schizochytrium* sp. microorganisms is disclosed (U.S. Patent No. 5,130,242) and additionally, a method for producing docosahexaenoic acid and eicosapentaenoic acid using *Thraustochytrium* sp. PTA10212 which is a Thraustochytrid-based *Thraustochytrium* sp. microorganism is disclosed.

[PRIOR ART]

[PATENT DOCUMENT]

(Patent document 1) U.S. Patent Publication US 5130242 A

[DISCLOSURE]

[TECHNICAL PROBLEM]

One embodiment of the present application provides a novel *Schizochytrium* sp. microalgae. In one specific embodiment, the novel *Schizochytrium* sp. microalgae may be *Schizochytrium* sp. CD01-1003 strain that is a microalgae deposited under accession number KCTC15201BP, which produces bio-oil comprising a high concentration of DHA (Docosahexaenoic acid, 22:6),

Another embodiment of the present application provides a microbial product(agent) for producing DHA (Docosahexaenoic acid, 22:6) comprising the *Schizochytrium* sp. strain or a culture solution thereof as an active ingredient.

Other embodiment of the present application provides biomass derived from the *Schizochytrium* sp. strain, comprising the *Schizochytrium* sp. strain, a culture solution of the strain, a dry matter of the culture solution, or a lysate of the dry matter.

Other embodiment of the present application provides a feed composition or a food composition comprising the biomass derived from the *Schizochytrium* sp. strain, or a concentrate, dry matter or extract of the biomass.

Other embodiment of the present application provides a method for producing biomass or bio-oil derived from the *Schizochytrium* sp. strain.

[TECHNICAL SOLUTION]

Each description and embodiments disclosed in the present application can be also be applied to each other description and embodiments. In other words, all combinations of various elements disclosed in the present application fall within the scope of the present application. In addition, it cannot be considered that the scope of the present application is limited by the specific description described below. Furthermore, those skilled in the art can recognize or confirm many equivalents to the specific embodiments of the present application disclosed in the present application using only common experiments. In addition, these equivalents are intended to be included in the present application.

One embodiment of the present application provides a novel *Schizochytrium* sp. microalgae.

The term used in the present description, *"Schizochytrium* sp." is one of genus names belonging to the family Thraustochytriaceae of the order Thraustochytriales, and can be interchangeably used with the term "genus *Schizochytrium"*. In addition, the term "microalgae" means an organism that cannot be seen with naked eyes and can only be seen with a microscope and live freely floating in water among plants which photosynthesize with chlorophyll, and is also called phytoplankton.

The novel *Schizochytrium* sp. strain may be a mutant strain obtained by mutating a *Schizochytrium* sp. strain which is a parent strain, and may be a strain with enhanced productivity of bio-oil and/or DHA compared to the parent strain.

The term used in the present description, "parent strain" means a strain before mutagenesis, and may be interchangeably used with "wild-type strain".

The parent strain may be a wild-type *Schizochytrium* sp. strain, and specifically, may be a wild-type *Schizochytrium* sp. CD01-1821 strain, but not limited thereto.

The term used in the present description, "productivity" means an ability capable of producing bio-oil or DHA (Docosahexaenoic acid, 22:6), and may be interchangeably used with "production capacity", and it may be confirmed by measuring the content of bio-oil or DHA comprised in a culture solution or strain-derived biomass after culturing a strain producing bio-oil or DHA.

The method of mutation may be performed by various means known in the art, and one method of physical or chemical mutagenesis methods may be used. For example, as a physical mutation method suitable for the present invention, a method for irradiating gamma rays or ultraviolet rays may be used, but not limited thereto. In addition, as a chemical mutation method, N-methyl-N'-nitro-N-nitrosoguanidine (NTG), diepoxybutane, ethylmethane sulfonate, mustard compounds, hydrazine and nitrous acid may be used, but not limited thereto.

The mutation for producing the mutant strain may be by a method for irradiating radiation.

The radiation for inducing the mutation may be gamma rays or ultraviolet rays, and preferably, gamma rays.

The radiation may be 6.0 kGy to 8.0 kGy, 6.5 kGy to 8.0 kGy, 6.7 kGy to 8.0 kGy, 6.9 kGy to 8.0 kGy, 6.5 kGy to 7.5 kGy, 6.7 kGy to 7.5 kGy, 6.9 kGy to 7.5 kGy, 6.5 kGy to 7.3 kGy, 6.7 kGy to 7.3 kGy, 6.9 kGy to 7.3 kGy, 6.5 kGy to 7.1 kGy, 6.7 kGy to 7.1 kGy, 6.9 kGy to 7.1 kGy, or 7.0 kGy per hour.

In the present application, as one embodiment, mutation was generated by irradiating gamma rays to the wild-type *Schizochytrium* sp. CD01-1821 strain, and a strain with enhanced productivity of oil containing polyunsaturated fatty acids among the mutant strains, and this was named *Schizochytrium* sp. CD01-1003, and deposited to an international depository authority, Korea Research Institute of Bioscience and Biotechnology Korea Collection for Type Culture (KCTC) under Budapest treaty on November 21, 2022, and given Accession number KCTC15201BP.

Therefore, in the present description, the novel *Schizochytrium* sp. microalgae may be *Schizochytrium* sp. CD01-1003 strain, which is a microalgae deposited under accession number KCTC15201BP.

In addition, the wild-type *Schizochytrium* sp. strain may have the 18s rRNA nucleotide sequence of SEQ ID NO: 1, but not limited thereto. For example, the *Schizochytrium* sp. microalgae may have 18S rRNA consisting of the nucleotide sequence showing sequence identity of 80% or more, 85% or more, 90% or more, 95% or more, 98% or more, or 99% or more to the nucleotide sequence of SEQ ID NO: 1, but not limited thereto.

The genomic DNA of the *Schizochytrium* sp. CD01-1003 strain may be one in which 36 nucleotides are deleted from the genomic DNA of the *Schizochytrium* sp. CD01-1821 strain, which is the parent strain.

The genomic DNA of the *Schizochytrium* sp. CD01-1003 strain may comprise a nucleotide sequence in which 36 nucleotides are deleted from the nucleotide sequence of SEQ ID NO: 4 of the *Schizochytrium* sp. CD01-1821 strain, which is the parent strain.

The nucleotide sequence in which 36 nucleotides are deleted from the nucleotide sequence of SEQ ID NO: 4 may consist of SEQ ID NO: 5.

The *Schizochytrium* sp. CD01-1003 strain may be identified with a primer set consisting of nucleotide sequences of SEQ ID NO: 6 and SEQ ID NO: 7.

In one specific embodiment of the present invention, as a result of comparing the whole genome sequences of the novel *Schizochytrium* sp. mutant strain of the present invention and the parent strain, it was confirmed that the *Schizochytrium* sp. CD01-1003 strain had a part that nucleotides of 36 bp are deleted in the genome of the wild-type CD01-1821 strain, which is a parent strain, and as a result of PCR with a primer set capable of amplifying DNA fragments comprising the corresponding part using the same, it was confirmed that the parent strain, the wild-type strain, and the *Schizochytrium* sp. CD01-1003 strain could be identified (distinguished).

Therefore, the primer set consisting of nucleotide sequences of SEQ ID NO: 6 and SEQ ID NO: 7 capable of amplifying DNA fragments comprising a nucleotide sequence in which 36 nucleotides are deleted from the nucleotide sequence of SEQ ID NO: 4 can be used as a use for selecting the *Schizochytrium* sp. CD01-1003 strain.

The term used in the present description, "docosahexaenoic acid (DHA)" is one of polyunsaturated fatty acids having the chemical formula of $C_{22}H_{32}O_2$, and corresponds to omega-3 fatty acids together with $\alpha$-linolenic acid (ALA) and eicosapentaenoic acid (EPA), and the common name is cervonic acid, and it may be represented by an abbreviation as 22:6 n-3.

The novel *Schizochytrium* sp. strain can produce bio-oil comprising a high concentration of DHA (Docosahexaenoic acid, 22:6).

The *Schizochytrium* sp. microalgae may produce and/or comprise DHA of 25 % by weight or more, 28 % by weight or more, 30 % by weight or more based on the total weight of fatty acids. For example, the *Schizochytrium* sp. microalgae may produce and/or comprise DHA of 25 to 50 % by weight, 25 to 45 % by weight, 25 to 40 % by weight, 25 to 35 % by weight, 28 to 50 % by weight, 28 to 45 % by weight, 28 to 40 % by weight, 28 to 35 % by weight, 28 to 34 % by weight, 28 to 33 % by weight, 28 to 32 % by weight, 28 to 31 % by weight, 30 to 50 % by weight, 30 to 45 % by weight, 30 to 40 % by weight, 30 to 35 % by weight, 30 to 34 % by weight, 30 to 33 % by weight, 30 to 32 % by weight, or 30 to 31 % by weight based on the total weight of fatty acids.

The productivity of bio-oil of the *Schizochytrium* sp. microalgae may be 44 to 54 g/L·day, 45 to 54 g/L·day, 46 to 54 g/L·day, 47 to 54 g/L·day, 44 to 53 g/L·day, 45 to 53 g/L·day, 46 to 53 g/L·day, 47 to 53 g/L·day, 44 to 52 g/L·day, 45 to 52 g/L·day, 46 to 52 g/L·day, 47 to 52 g/L·day, 44 to 51 g/L·day, 45 to 51 g/L·day, 46 to 51 g/L·day, 47 to 51 g/L·day, 44 to 50 g/L·day, 45 to 50 g/L·day, 46 to 50 g/L·day, 47 to 50 g/L·day, 44 to 49 g/L·day, 45 to 49 g/L·day, 46 to 49 g/L·day, 47 to 49 g/L·day, 44 to 48 g/L·day, 45 to 48 g/L·day, 46 to 48 g/L·day, or 47 to 48 g/L·day.

The productivity of DHA of the *Schizochytrium* sp. microalgae may be 12.5 to 16.5 g/L·day, 13 to 16.5 g/L·day, 13.5 to 16.5 g/L·day, 14 to 16.5 g/L·day, 14.5 to 16.5 g/L·day, 12.5 to 16 g/L·day, 13 to 16 g/L·day, 13.5 to 16 g/L·day, 14 to 16 g/L·day, 14.5 to 16 g/L·day, 12.5 to 15.5 g/L·day, 13 to 15.5 g/L·day, 13.5 to 15.5 g/L·day, 14 to 15.5 g/L·day, 14.5 to 15.5 g/L·day, 12.5 to 15 g/L·day, 13 to 15 g/L·day, 13.5 to 15 g/L·day, 14 to 15 g/L·day, or 14.5 to 15 g/L·day.

Another aspect of the present application provides a microbial product(agent) for producing DHA (Docosahexaenoic acid, 22:6) comprising the *Schizochytrium* sp. strain or a culture solution thereof as an active ingredient.

The microbial product(agent) may comprise the *Schizochytrium* sp. CD01-1003 strain as an active ingredient, and may be effectively used for production of DHA.

Other aspect of the present application provides biomass or bio-oil derived from a *Schizochytrium* sp. strain, comprising the *Schizochytrium* sp. strain, a culture of the strain, a dry matter of the culture, or a lysate of the dry matter.

Matters for the *Schizochytrium* sp. strain are the same as described above.

The term used in the present description, "biomass" means an organism such as a plant, an animal, a microorganism or the like, usable as chemical energy, that is, an energy source of bio-energy, and ecologically, also means a weight or energy amount of a specific organism present in a unit of time and space. In addition, the biomass includes compounds secreted by cells, but not limited thereto, and may contain cells and/or intracellular contents as well as extracellular substances. In the present application, the biomass may be a product produced by culturing or fermenting the *Schizochytrium* sp. strain itself, a culture thereof, a dry matter thereof, a lysate thereof, or the microalgae, or may be a concentrate or dry matter of the biomass, but not limited thereto.

"Culture" of the *Schizochytrium* sp. microalgae refers to a product produced by culturing the microalgae, and specifically, it may be a culture solution comprising the microalgae, or a culture filtrate in which the microalgae is removed from the culture solution, but not limited thereto. "Dry matter" of the *Schizochytrium* sp. microalgal culture may be one in which moisture is removed from the microalgal culture, and for example, it may be in a form of dry microbial cells of the microalgae, but not limited thereto. In addition, "lysate" collectively refers to a result of lysing a dry matter in which moisture is removed from the microalgal culture, and for example, it may be a dry microbial cell powder, but not limited thereto. The culture of the *Schizochytrium* sp. microalgae may be produced according to a culture method known in the art by inoculating the microalgae in a microalgal culture medium, and the dry matter of the culture and lysate thereof may be also produced according to a treatment or drying method of a microalgae or culture solution known in the art.

The biomass derived from the *Schizochytrium* sp. microalgae may comprise crude fat of 61 to 85 % by weight, 61 to 80 % by weight, 61 to 75 % by weight, 61 to 72 % by weight, 65 to 85 % by weight, 65 to 80 % by weight, 65 to 75 % by weight, 65 to 72 % by weight, 68 to 85 % by weight, 68 to 80 % by weight, 68 to 75 % by weight, 68 to 72 % by weight, 70 to 85 % by weight, 70 to 80 % by weight, 70 to 75 % by weight, or 70 to 72 % by weight based on the total weight of biomass.

The term used in the present description, "crude fat content" may mean a content of intracellular oil, and may be interchangeably used with "crude fat amount" or "total lipid" or "total fatty acid" or "TFA" or "oil content".

In one specific embodiment of the present invention, the *Schizochytrium* sp. CD01-1003 strain or *Schizochytrium* sp. CD01-1003 strain-derived biomass has a high crude fat content, so they are suitable for oil production, and therefore, the *Schizochytrium* sp. CD01-1003 strain may have high oil productivity.

The *Schizochytrium* sp. CD01-1003 strain-derived biomass may comprise DHA of 25 % by weight or more, 28 % by weight or more, 30 % by weight or more, based on the total weight of fatty acids. For example, the *Schizochytrium* sp. microalgae may comprise DHA of 25 to 50 % by weight, 25 to 45 % by weight, 25 to 40 % by weight, 25 to 35 % by weight, 28 to 50 % by weight, 28 to 45 % by weight, 28 to 40 % by weight, 28 to 35 % by weight, 28 to 34 % by weight, 28 to 33 % by weight, 28 to 32 % by weight, 28 to 31 % by weight, 30 to 50 % by weight, 30 to 45 % by weight, 30 to 40 % by weight, 30 to 35 % by weight, 30 to 34 % by weight, 30 to 33 % by weight, 30 to 32 % by weight, or 30 to 31 % by weight based on the total weight of fatty acids.

Other aspect of the present application provides a composition comprising the *Schizochytrium* sp. CD01-1003 strain, a culture of the strain, a dry matter of the culture, or a lysate of the dry matter.

The composition may comprise biomass, bio-oil, or a combination thereof, which is derived from the *Schizochytrium* sp. strain.

Other aspect of the present application provides a feed composition comprising biomass derived from the *Schizochytrium* sp. CD01-1003 strain, or a concentrate or dry matter of the biomass.

The *Schizochytrium* sp. CD01-1003 strain, biomass, culture of the strain, dry matter of the culture, and lysate of the dry matter are described above.

The concentrate or dry matter of the biomass may be produced according to a treatment, concentration or drying method of microbial biomass known in the art.

The term used in the present description, "bio-oil" means oil obtained from biomass by biological, thermochemical and physicochemical extraction processes, and the bio-oil produced in the present application may contain polyunsaturated fatty acids, and specifically, it may contain DHA, but not limited thereto.

In the present description, the bio-oil may comprise an extract of biomass.

As a method for producing the bio-oil extract, according to a method for lysing or dissolving cell membrane or cell wall components, a method for using an enzyme such as protease, cellulase, pectinase, or chitinase or the like, a method for lysing cell membrane or cell wall components physically using a homogenizer, a ultrasonicator, bead treatment, or the like, a method for extracting by directly adding a solvent and penetrating intracellularly, a solvent-free extraction process for isolation through a centrifuging process after various lysing processes, or the like may be used, but not limited thereto.

The composition may be in a form of solution, powder, or suspension, but not limited thereto. The composition may be for example, a food composition, a feed composition or a feed additive composition.

The term used in the present description, "feed composition" refers to a feed fed to an animal. The feed composition refers to a substance supplying organic or inorganic nutrients required for maintaining lives of animals or producing meat, milk, and the like. The feed composition may additionally comprise nutritional components required for maintaining lives of animals or producing meat, milk, and the like. The feed composition may be produced as various forms of feeds known in the art, and specifically, it may comprise a concentrated feed, a crude feed and/or a special feed.

The term used in the present description, "feed additive" includes substances to be added to a feed for a purpose of various effects such as supplementation of nutrients and prevention of weight loss, enhancement of digestibility of fibers in a feed, improvement of milk quality, prevention of reproductive failure and enhancement of a fertility rate, prevention of summer high temperature stress, and the like. The feed additive of the present application corresponds to a supplementary feed under Control of Livestock and Fish Feed Act, and may further comprise a mineral matter preparation such as sodium bicarbonate, bentonite, magnesium oxide, a composite mineral, and the like, a mineral preparation which is a trace amount of trace matter such as zinc, copper, cobalt, selenium and the like, a vitamin preparation such as carotene, vitamin E, vitamins A, D, E, nicotinic acid, vitamin B complexes and the like, a protected amino acid preparation such as methionine, lysine and the like, a protected fatty acid preparation such as fatty acid calcium salts, and the like, a living cell and a yeast preparation such as probiotics (lactic acid bacterial preparation), yeast cultures, fungal fermented matters and the like.

The term used in the present description, "food composition" includes all forms such as functional food, nutritional supplement, health food and food additives and the like, and the food composition in the above form may be produced in various forms according to a common method known in the art.

The composition of the present application may further comprise grains, for example, pulverized or crushed wheat, oats, barley, corn and rice; vegetable protein feed, for example, feed comprising soybeans and sunflower as main components; animal protein feed, for example, blood meal, meat meal, bone meal and fish meal; sugar and dairy products, for example, dry components composed of various kinds of powdered milk and whey powder, and the like, and in addition thereto, it may further comprise a dietary supplement, a digestion and absorption enhancer, a growth promoting agent, and the like.

The composition of the present application may be administered alone or administered in combination with other feed additive among edible carriers into an animal. **In** addition, the composition can be administered easily into an animal as top dressing or directly mixed in feed, or an oral formulation separate to feed. When the composition is administered separately to feed, it can be produced as an immediate release or sustained-release formulation, in combination with a pharmaceutically acceptable edible carrier as well known in the art. Such an edible carrier may be a solid or liquid, for example, corn starch, lactose, sucrose, soybean flake, peanut oil, olive oil, sesame oil and propylene glycol. When a solid carrier is used, the composition may be a tablet, a capsule, a troche, or a lozenge, or top dressing in a microdispersible form. When a liquid carrier is used, the composition may be a formulation in a gelatin soft capsule, or syrup or suspension, emulsion, or solution.

The composition of the present application may contain for example, a preservative, stabilizer, wetting agent or emulsifier, cryoprotectant, or excipient, or the like. The cryoprotectant may be at least one selected from the group consisting of glycerol, trehalose, maltodextrin, nonfat dry milk and starch.

The preservative, stabilizer or excipient may be comprised in the composition in a sufficiently effective dose for reducing deterioration of the *Schizochytrium* sp. microalgae comprised in the composition. In addition, the cryoprotectant may be comprised in a sufficiently effective dose for reducing deterioration of the *Schizochytrium* sp. microalgae comprised in the composition, when the composition is in a dried condition.

The composition may be used by adding to feed of an animal by immersing, spraying or mixing.

The composition of the present application may be applied to a number of animal diets including mammals, birds, fish, crustaceans, cephalopods, reptiles, and amphibians, but not limited thereto. For example, the mammals may include pigs, cows, sheep, goats, laboratory rodents, or pets, and the birds may include poultry, and the poultry may include chickens, turkeys, ducks, geese, pheasant or quail, or the like, but not limited thereto. In addition, the fish may include commercial farmed fish and their fry, aquarium fish, and the like, and the crustaceans may include shrimp, barnacles, and the like, but not limited thereto. Furthermore, the composition may be also applied to a diet of a rotifer, which is zooplankton.

Other aspect of the present application provides a method for producing biomass derived from a *Schizochytrium* sp. strain, comprising culturing *Schizochytrium* sp. CD01-1003 strain; and recovering biomass from the strain, a culture of the strain, a dry matter of the culture, or a lysate of the dry matter.

The *Schizochytrium* sp. strain, biomass, culture of the strain, dry matter of the culture, and lysate of the dry matter are the same as described above.

The term used in the present description, "culture" means growing the strain under an appropriately controlled environmental condition. The culture process of the present application may be conducted according to an appropriate medium and a culture condition known in the art. Such a culture process may be easily adjusted and used by those skilled in the art depending on the strain to be selected.

Specifically, the culture of the *Schizochytrium* sp. strain of the present application may be performed under a heterotrophic condition, but not limited thereto.

The term used in the present description, "heterotroph" is a nutritional method which depends on an organic matter obtained from outside the body for an energy source or nutrient source, and is a term corresponding to autotroph, and it may be interchangeably used with the term 'dark culture'.

The culturing the *Schizochytrium* sp. strain, is not particularly limited thereto, but may be performed by a known batch culture method, a continuous culture method, a fed-batch culture method, and the like. For the medium used for culture of the microalgae of the present application and other culture conditions, any one may be used without particular limitations as long as it is a common medium used for culture of microalgae. Specifically, the microalgae of the present application may be cultured in a common medium containing appropriate carbon sources, nitrogen sources, phosphorus sources, inorganic compounds, amino acids and/or vitamins and the like by adjusting temperature, pH and the like.

Specifically, appropriate pH (for example, pH 5 to 9, specifically, pH 6 to 8, most specifically, pH 6.8) may be adjusted using a basic compound (e.g.: sodium hydroxide, potassium hydroxide, or ammonia) or an acidic compound (e.g.: phosphoric acid or sulfuric acid), but not limited thereto.

Moreover, oxygen or oxygen-containing gas may be injected into a culture in order to maintain an aerobic condition of the culture, or no gas may be injected or nitrogen, hydrogen or carbon dioxide gas may be injected to the culture in order to anaerobic and non-aerobic conditions, but not limited thereto.

In addition, the culturing temperature may be maintained at 20 to 45°C or 25 to 40°C, and culturing may be performed for about 10 to 160 hours, but not limited thereto. Furthermore, during culturing, bubble generation can be inhibited using an antifoaming agent such as fatty acid polyglycol ester, but not limited thereto.

The carbon source comprised in the medium used in the culturing the *Schizochytrium* sp. microalgae, may be at least one selected from the group consisting of glucose, fructose, maltose, galactose, mannose, sucrose, arabinose, xylose and glycerol, but not limited thereto as long as it is a carbon source used for culturing microalgae.

The nitrogen source comprised in the medium used in the culturing the *Schizochytrium* sp. microalgae, may be i) at least one organic nitrogen source selected from the group consisting of yeast extract, beef extract, peptone and tryptone, or ii) at least one inorganic nitrogen source selected from the group consisting of ammonium acetate, ammonium nitrate, ammonium chloride, ammonium sulfate, sodium nitrate, urea and MSG (Monosodium glutamate), but not limited thereto as long as it is a nitrogen source used for culturing microalgae.

To the medium used in the culturing the *Schizochytrium* sp. microalgae, potassium dihydrogen phosphate, dipotassium hydrogen phosphate, sodium-containing salts corresponding thereto, and the like may be individually comprised or comprised in combination as a phosphorus source, but not limited thereto.

The recovering biomass from the microalgae, culture of the microalgae, dry matter of the culture, or lysate of the dry matter may collect desired biomass using an appropriate method known in the art. For example, centrifugation, filtration, anion exchange chromatography, crystallization and HPLC and the like may be used, and it may further comprise a purification process.

Other aspect of the present application provides a method for producing bio-oil derived from a *Schizochytrium* sp. microalgae, comprising culturing *Schizochytrium* sp. CD01-1003 microalgae; and recovering lipids from the microalgae, a culture of the microalgae, a dry matter of the culture, or a lysate of the dry matter.

The culturing the *Schizochytrium* sp. microalgae, bio-oil, culture of the microalgae, dry matter of the culture, and lysate of the dry matter, and the culturing the microalgae are the same as described above.

The recovering lipids from the microalgae, culture of the microalgae, dry matter of the culture, or lysate of the dry matter may collect desired lipids using an appropriate method known in the art. For example, centrifugation, filtration, anion exchange chromatography, crystallization and HPLC and the like may be used, and it may further comprise a purification process.

For example, lipids and lipid derivatives such as fat aldehyde, fat alcohol and hydrocarbon (for example, alkane) may be extracted with a hydrophobic solvent. The lipids and lipid derivatives may be also extracted using a method such as liquefaction, oil liquefaction and supercritical $CO_2$ extraction and the like. In addition, a known method for recovering microalgal lipids includes for example, a method of i) collecting cells by centrifugation and washing with distilled water, and then drying by freeze-drying, and ii) pulverizing obtained cell powders and then extracting lipids with n-hexane (Miao, X and Wu, Q, Biosource Technology (2006) 97:841-846).

[ADVANTAGEOUS EFFECTS]

The *Schizochytrium* sp. strain which is a bio-oil high-producing a microalgae comprising a high concentration of DHA of the present invention has a high crude fat content in produced biomass, so the productivity of bio-oil is high, and has a characteristic of comprising intracellular docosahexaenoic acid in a high content. Accordingly, the strain itself, or biomass produced by culture and fermentation of the strain, and a concentrate and an extract thereof can be usefully used as a feed composition or a food composition.

[BRIEF DESCRIPTION OF THE DRAWINGS]

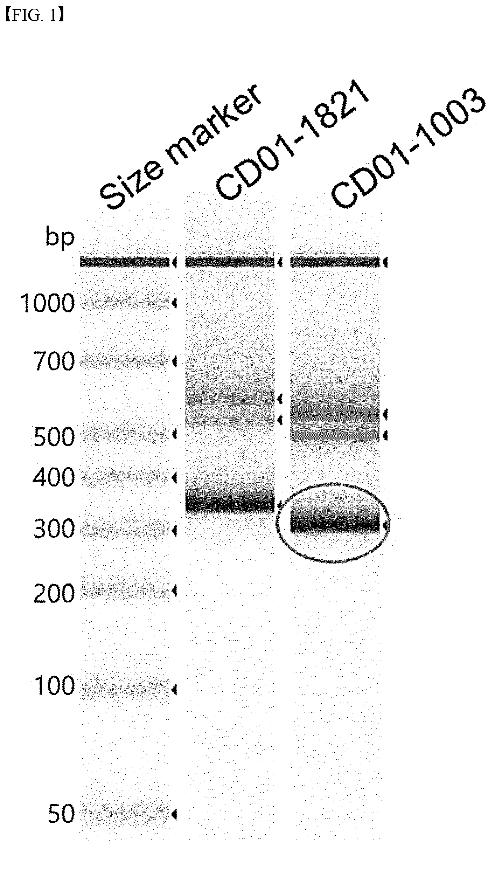

FIG. 1 is a drawing which confirms the sizes of amplified DNA fragments of the wild-type CD01-1821 strain and mutant CD01-1003 strain, after performing PCR using a primer set which amplifying DNA fragments comprising a base sequence mutated by deleting 36 bp from the mutant CD01-1003 strain compared to the wild-type CD01-1821 strain.

[MODE FOR INVENTION]

Hereinafter, the present invention will be described in more detail by examples. However, these examples are intended to illustratively describe one or more specific examples, but the scope of the present invention is not limited by these examples.

**Example 1. Isolation of Thraustochytrid-based microalgae**

In order to isolate thraustochytrid-based microalgae, environmental samples in a form of seawater, leaves and sediment were collected from a total of 40 areas in Korean west coastal areas, Seocheon, Gunsan, Buan and Yeonggwang coastal areas. Sampling was conducted focusing on specific areas where organic sediments were developed and observed, and the collected environmental samples were transported to a laboratory environment within 7 days to remove other contaminants such as bacterial microorganisms and fungi, protists, and the like excluding thraustochytrid-based microalgae to be isolated. Through continuous microscopy, focusing on samples which exhibited a unique morphology of thraustochytrid-based microalgae and formed zoospores which could be observed within the life cycle, or an ectoplasmic network was created at the development stage in, thraustochytrid-based microalgae cells were isolated. As the isolation and culture medium used in the isolation process, modified YEP medium (Yeast extract 0.1 g/L, peptone 0.5 g/L, $MgSO_4 \cdot 7H_2O$ 2 g/L, sea salt 50 g/L, $H_3BO_3$ 5.0 mg/L, $MnCl_2$ 3.0 mg/L, $CuSO_4$ 0.2 mg/L, $NaMo_4 \cdot 2H_2O$ 0.05 mg/L, $CoSO_4$ 0.05 mg/L, $ZnSO_4 \cdot 7H_2O$ 0.7 mg/L, agar 15 g/L) was used. Through several times of isolation and subculture processes, pure isolated colonies in which contaminants were removed could be obtained, and the isolated colonies were passed through a contaminant control and removal process again in a solid medium comprising an antibiotic cocktail mix solution (Streptomycin sulfate 0-50 mg/L, Ampicillin 0-30 mg/L, Penicillin G 0-30 mg/L, Kanamycin sulfate 0-30 mg/L) to obtain pure separable colonies.

**Example 2. Evaluation of culture of isolated microalgae and selection of excellent strains**

Culture evaluation was performed for the pure isolated colonies in Example 1, and through this, excellent strains were selected.

Specifically, the pure isolated colonies in Example 1 were cultured using modified GYEP medium (glucose 5 g/L, glycerol 5 g/L, yeast extract 0.1 g/L, peptone 0.5 g/L, $MgSO_4 \cdot 7H_2O$ 2 g/L, sea salt 50 g/L, $H_3BO_3$ 5.0 mg/L, $MnCl_2$ 3.0 mg/L, $CuSO_4$ 0.2 mg/L, $NaMo_4 \cdot 2H_2O$ 0.05 mg/L, $CoSO_4$ 0.05 mg/L, $ZnSO_4 \cdot 7H_2O$ 0.7 mg/L) in a 250 mL flask under conditions of 10-35°C, 100-200 rpm for about 2 days. Based on the result of the conducted culture, 29 kinds of microalgae, which could grow under a temperature condition of 30°C or higher and had an excellent growth rate, and could secure microbial cell mass, were selected. Culture of modified GYEP medium comprising glucose of 30 g/L as a carbon source and culture conditions of 30°C, 150 rpm and a 500 ml flask scale was carried out for the selected microalgal strains for 2 days. After confirming that all the added carbon sources were consumed in a culture environment for 2 days, the whole culture solution was collected and dried in a 60°C dry oven overnight to obtain biomass.

In order to analyze the contents of lipids and polyunsaturated fatty acids of the cultured microalgal microbial cells, the following method was used, and the microalgae-derived fatty acid containing oil using the dry microbial cells was measured by the following method. 8.3 M hydrochloric acid solution (HCl) was added to the dry microbial cells 5 g, and the cell walls of the microalgal microbial cells were hydrolyzed at 80°C, and then ethyl ether 30 mL and petroleum ether 20 mL were added and mixed for 30 seconds, and then a centrifuging process was repeated 3 times or more. The isolated solvent layer was collected and moved to a round flask of which weight was measured previously, and then the solvent was removed through nitrogen purging, and cooled and weighed in a desiccator. The weight of the dried oil was measured with a value of subtracting the weight of the empty flask from the flask weight after drying, and the total oil content was calculated. The docosahexaenoic acid (DHA) content comprised in the oil was shown by pretreating with methanolic 0.5N NaOH and 14% trifluoroborane methanol ($BF_3$) and measuring by gas chromatography.

[Equation 1]

$$\text{Total oil content (\%)} = (*\text{oil g/dry microbial cell mass g}) \times 100$$

*oil g: Flask weight after acid hydrolysis and solvent removal - empty flask weight

"Biomass" of Table 1 below means a concentration of microbial cells in a culture solution, and can be interchangeably used with DCW (dry cell weight) of Table 2 below.

---

EP 4 560 005 A1

[Table 1]

| | Total oil (%/Biomass) | Fatty acid content (%/TFA) | |
|---|---|---|---|
| | | DHA | EPA |
| 1811 | 40.01 | 19.46 | 1.72 |
| 1812 | 36.03 | 17.90 | 1.80 |
| 1813 | 34.21 | 19.40 | 2.10 |
| 1814 | 38.67 | 18.90 | 1.98 |
| 1815 | 28.89 | 17.90 | 2.92 |
| 1816 | 23.84 | 17.64 | 3.38 |
| **1821** | **31.53** | **57.25** | **0.71** |
| **1822** | **43.18** | **58.68** | **0.53** |
| 1831 | 29.80 | 29.38 | 1.98 |
| 1832 | 31.50 | 33.35 | 1.61 |
| 1833 | 22.36 | 37.26 | 2.83 |
| 1834 | 23.44 | 36.12 | 2.22 |
| 1835 | 39.03 | 29.85 | 0.70 |
| 1836 | 21.73 | 35.77 | 2.76 |
| 1837 | 18.39 | 33.30 | 2.96 |
| 1838 | 19.81 | 38.77 | 2.76 |
| 1839 | 20.76 | 37.91 | 3.06 |
| 18310 | 20.65 | 39.93 | 2.85 |
| 18311 | 22.96 | 40.17 | 2.70 |
| 18312 | 18.26 | 39.27 | 3.31 |
| 18313 | 21.99 | 36.68 | 2.69 |
| 1841 | 26.33 | 19.40 | 1.06 |
| 1842 | 35.53 | 21.75 | 1.38 |
| 1843 | 34.83 | 18.67 | 1.02 |
| 1844 | 29.74 | 23.92 | 1.65 |
| 1845 | 33.23 | 19.89 | 0.89 |
| 1846 | 28.23 | 20.63 | 1.13 |
| 1847 | 31.37 | 18.56 | 0.91 |
| 1848 | 30.51 | 42.74 | 1.06 |
| 1849 | 29.19 | 20.46 | 1.50 |
| 18410 | 24.63 | 25.17 | 1.84 |
| 18411 | 21.93 | 25.75 | 1.96 |

(In the table, TFA means total fatty acids, and can be interchangeably used with crude fat content or crude fat amount or total lipids.)

As a result, as shown in Table 1, it was shown that the intracellular DHA content of 2 kinds of strains of CD01-1821 and CD01-1822 was very high as 50% or more.

As the fatty acid analysis, culture evaluation was performed in a 5L scale incubator for 2 kinds of strains of CD01-1821 and CD01-1822 with an excellent intracellular DHA content. Seed culture was conducted using sterilized MJW02 medium (glucose 30 g/L, $MgSO_4 \cdot 7H2O$ 3.0 g/L, $Na_2SO_4$ 15 g/L, NaCl 0.8 g/L, yeast extract 1.0 g/L, $MSG \cdot 1H_2O$

1.0 g/L, NaNO$_3$ 1.0 g/L, KH$_2$PO$_4$ 0.8 g/L, K$_2$HPO$_4$ 1.5 g/L, CaCl$_2$ 0.5 g/L, vitamin mixed solution 10 ml/L) in a 500 mL flask under conditions of 30°C, 150 rpm for about 24 hours. The seed cultured flask was aliquoted and inoculated in a 5L incubator. Glucose carbon sources of 28% compared to the total culture solution were supplied and culture was carried out for about 72 hours, and culture was performed in sterilized MJW02 medium under conditions of the culture environment 30°C, 500 rpm, 1.5 vvm, pH 5-8.

[Table 2]

|  | *Schizochytrium* sp. CD01-1821 | *Schizochytrium* sp. CD01-1822 |
|---|---|---|
| **Culture time (hr)** | 71.5 | 71.5 |
| **O.D (680nm)** | 154.2 | 103 |
| **DCW (g/L)** | 139.5 | 103 |
| **Crude fat amount (%)** | 58.6 | 49.7 |

As a result, as shown in Table 2, it was confirmed that the CD01-1821 strain was easier in a scale-up process, as it had higher biomass total production and crude fat amount than the CD01-1822 strain in the same fermentation condition. Therefore, the CD01-1821 strain was selected and used for strain sequence identification and additional strain development.

**Example 3. Confirmation of culture characteristics of CD01-1821 strain under complex carbon sources**

In heterotrophic microorganism-based fermentation, glucose components are mainly used as a raw material of a carbon source. At this time, glucose is a monosaccharide in a purified form by 90% or more, and it costs more during industrial scale fermentation compared to other carbon source raw material components. In order to use inexpensive carbon source raw materials and secure price competitiveness through this, it is important to discover strains that can be cultured normally in carbon source components, which are not glucose in a purified form, and can be used during fermentation by microorganisms and are inexpensive.

Accordingly, fermentation culture evaluation in raw sugar using glucose, fructose or sucrose as a main ingredient was performed for the CD01-1821 strain selected in Example 2 and CJM01 (Granted Patent 10-2100650) strain, and culture characteristics were confirmed. Culture was performed in a 30L incubator, and experiments were conducted with a raw sugar lysate comprising glucose 450 g/L, a mixture of glucose 225 g/L and fructose 225 g/L, and glucose 225 g/L, fructose 220 g/L and sulfate 1.51 g/L as main carbon source components based on modified MJW02 medium, respectively. Culture conditions were set as same as conditions of 30°C, 500 rpm, 1.5 vvm, pH 5-8, and carbon sources of 35% of the total culture solution volume were supplied, respectively.

[Table 3]

|  | *Schizochytrium* sp. CD01-1821 | | | *Thraustochytrium* sp. CJM01 | | |
|---|---|---|---|---|---|---|
| **Carbon source** | Glucos e | Mixture of glucose + fructose | Sucrose lysate | Glucose | Mixture of glucose + fructose | Sucrose lysate |
| **Culture time (hr)** | 54.7 | 56.1 | 7.3 | 60 | 66.83 | 83.3 |
| **O.D (680nm)** | 169.3 | 143.7 | 177.9 | 152.7 | 180.4 | 155.7 |
| **DCW (g/L)** | 163 | 160 | 161 | 130 | 150 | 138 |
| **Crude fat amount (%)** | 60.7 | 60.1 | 60.3 | 61.2 | 61.7 | 59.3 |

As a result, as shown in Table 3, the CD01-1821 strain showed the total biomass production and crude fat amount at an equivalent level or more even in fermentation under a fructose mixture, not a glucose single component, or sucrose lysate medium condition. On the other hand, the CJM01 strain showed a diauxic growth form of dualized carbon source consumption and cell growth patterns as a sugar component in addition to the glucose component was added in the medium and showed a phenomenon that the total culture time was prolonged. Through the corresponding experiment, the CD01-1821 strain could be confirmed to have scaled-up fermentation possibility under a complex carbon source condition.

**Example 4. Identification of novel *Schizochytrium* sp. Strain CD01-1821**

For molecular biological identification of the microalgal strain CD01-1821 isolated and selected in Example 1 and Example 2, 18S rRNA gene sequence was analyzed.

Specifically, gDNA was extracted and isolated from colonies of the pure isolated microalgae CD01-1821, and then a PCR amplification reaction was performed using the primers for gene amplification of the 18s rRNA site of 18s-Fwd, LABY-ARev described in Table 4.

[Table 4]

| SEQ ID NO: | Primer | Sequence (5' - 3') |
|---|---|---|
| 2 | 18S-Fwd | AAC CTG GTT GAT CCT GCC AGT |
| 3 | LABY-ARev | GGG ATC GAA GAT Arev TAG |

The PCR reaction was performed by denaturation at 95°C for 5 minutes using a reaction solution containing taq polymerase, and then repeating denaturation at 95°C for 30 seconds, annealing at 50°C for 30 seconds, and polymerization at 72°C for 2 minutes 35 times, and then polymerization reaction at 72°C for 5 minutes. The reaction solution amplified through the PCR process was under electrophoresis in 1% agarose gel, and it was confirmed that DNA fragments of about 1000 bp in size were amplified, and base sequencing analysis was conducted. The corresponding sequence obtained as a result of analysis, through NCBI BLAST search, it was confirmed that it showed 95.11% homology to the 18S rRNA gene sequence of *Schizochytrium limacinum* strain OUC109 belonging to thraustochytride family-based microalgae, and showed 95.0% homology to the 18S rRNA gene sequence of *Schizochytrium* sp. strain LY-2012. Through this, it was confirmed that the isolated microalgae CD01-1821 was a novel *Schizochytrium* sp. strain, and this was named *Schizochytrium* sp. CD01-1821 strain, and deposited to Korea Research Institute of Bioscience and Biotechnology Korea Collection for Type Culture (KCTC) on August 23, 2021 and given accession number KCTC14660BP.

**Example 5. Development of novel *Schizochytrium* microalgal artificial mutant strain**

An artificial mutant strain was developed according to irradiation of gamma rays by the following method from the wild-type microalgal strain isolated in Example 4 *(Schizochytrium* sp. CD01-1821).

Specifically, the pure isolated *Schizochytrium* sp. CD01-1821 strain (KCTC14660BP) was cultured in modified-GYEP medium (glucose 10 g/L, yeast extract 1 g/L, peptone 1 g/L, $MgSO_4 \cdot 7H_2O$ 2 g/L, $H_3BO_3$ 5.0 mg/L, $MnCl_2$ 3.0 mg/L, $CuSO_4$ 0.2 mg/L, $NaMo_4 \cdot 2H_2O$ 0.05 mg/L, $CoSO_4$ 0.05 mg/L, $ZnSO_4 \cdot 7H_2O$ 0.7 mg/L) for about 24 hours to reach the early exponential phase, and then the culture solution sample was centrifuged to harvest microbial cells. The harvested microbial cells were suspended in 0.1M Phosphate Buffer Solution comprising NaCl 1.0% so that the number of cells was about $10^9$ cells/mL, and used for irradiation of gamma rays.

The gamma ray irradiation experiment was conducted in Korea Atomic Energy Research Institute, Advanced Radiation Technology Institute, and gamma rays at a dose of 7 kGY were irradiated. A microalgal culture solution sample to which gamma rays were irradiated, was cultured was cultured in GYEP medium comprising agar 20g/L and 2-butanol inhibiting fatty acid synthesis, after passing through a recovery process for 0/N in a dark room. Microalgal colonies grown during culture for about 2 weeks were selected and passaged under the same medium and culture environment conditions. Strains which could continuously grow between passages and had excellent colony growth were preferentially selected. In addition, colonies that were morphologically white and growing rapidly were selected. The selected colonies were pure isolated and cultured as a single cell line, and the corresponding strain was named CD01-1003 strain, and deposited to Korea Research Institute of Bioscience and Biotechnology Korea Collection for Type Culture (KCTC) on November 21, 2022, and given accession number KCTC15201BP.

**Example 6. Confirmation of culture characteristics of mutant *Schizochytrium* sp. CD01-1003 strain**

**Example 6-1. Culture of CD01-1821 strain and CD01-1003 strain**

In order to confirm culture characteristics of the wild-type *Schizochytrium* sp. CD01-1821 strain and mutant *Schizochytrium* sp. CD01-1003 strain, the wild-type *Schizochytrium* sp. CD01-1821 strain selected in Example 2 and the mutant *Schizochytrium* sp. CD01-1003 strain selected in Example 5 were under fermentation culture evaluation.

Specifically, for preliminary culture before the present culture of a 30 L scale, they were cultured in a shaking incubator at 30°C, 180 rpm for about 20 hours by inoculating them to GYEP medium comprising glucose of working volume 50 ml and 30 g/L in a 500 ml flask. The preliminary culture was inoculated in a 30 L fermenter comprising medium under the same condition and fermented and cultured in a total working volume 20 L. Under conditions of 30°C, 500 rpm, 0.5-1 vvm, pH 5-7, glucose corresponding to 20% of the working volume was continuously inputted and used for cell culture, and it was injected so that the glucose concentration at this time was maintained at a level of 20 g/L. Culture was terminated when all the glucose which was the supplied carbon source was consumed. Microbial cells in which the supernatant was removed after completing the culture were used for experiments for measurement of crude fat and fatty acid, and crude protein contents, and intracellular extraction and recovery.

**Example 6-2. Analysis of crude fat and fatty acid contents of culture samples of CD01-1003 mutant strain and wild-type CD01-1821 strain**

In order to analyze the contents of lipids and polyunsaturated fatty acids in cells of the wild-type *Schizochytrium* sp. CD01-1821 strain and the mutant *Schizochytrium* sp. CD01-1003 strain, an experiment was performed by the following method.

Specifically, 8.3 M hydrochloric acid solution (HCl) was added to each dry microbial cell 2 g, obtained in Example 6-1 and the cell walls of the microalgal microbial cells were hydrolyzed at 80°C, and then ethyl ether 30 mL and petroleum ether 20 mL were added and mixed for 30 seconds, and then a centrifuging process was repeated 3 times or more. The isolated solvent layer was collected and moved to a round flask of which weight was measured previously, and then the solvent was removed through nitrogen purging, and cooled and weighed in a desiccator. The weight of the dried oil was measured, and the total oil content was calculated. The DNA content comprised in the oil was pretreated with methanolic 0.5N NaOH and 14% trifluoroborane methanol ($BF_3$) and measured by gas chromatography. The measured result was shown in Table 5 below.

■ Total oil content (%, * oil g / dry microbial cell mass g x 100)

* oil g: Flask weight after acid hydrolysis and solvent removal - empty flask weight

[Table 5]

|  | *Schizochytrium* sp. CD01-1821 | *Schizochytrium* sp. CD01-1003 |
|---|---|---|
| **Culture time (hr)** | 54.7 | 56.7 |
| **O.D (680nm)** | 169.3 | 198.6 |
| **DCW (g/L)** | 163 | 157.7 |
| **Crude fat productivity** | 43.41 g/L ·d | 47.86 g/L ·d |
| **C22:6 n-3 (DHA) productivity** | 12.11 g/L ·d | 14.54 g/L ·d |
| **Crude fat amount in biomass (%)** | 60.7 | 71.7 |
| **Fatty acids in biomass (%)** | | |
| **- C22:6 n-3 (DHA)** | 27.9 | 30.4 |

As a result, as shown in Table 5, both the wild-type CD01-1821 strain and the mutant CD01-1003 strain consumed all the supplied carbon source, glucose, within about 60 hours, and produced biomass at a level of about 160 g/L. In particular, in the CD01-1003 strain, 71.7% of the produced biomass consisted of crude fat components, and 30% or more of the biomass comprised a high content of omega 3 components of DHA (C22:6 n-3). In addition, the crude fat productivity of the CD01-1003 strain was shown as 47.86 g/L·d, and therefore, it was confirmed that it had more excellent crude fat productivity than the wild-type strain, and the C22:6 n-3 (DHA) productivity was shown as 14.54 g/L·d, and therefore, it was confirmed that it had higher C22:6 n-3 (DHA) productivity compared to the wild-type strain.

**Example 7. Derivation of distinguishing markers of CD01-1003 mutant strain and wild-type CD01-1821 strain**

PCR markers were produced by finding a sequence mutated in the CD01-1003 strain by the following method, by comparing the whole genome sequence of the wild-type CD01-1821 strain and mutant CD01-1003 strain.

Specifically, as a result of confirming the whole genome sequence of the mutant CD01-1003 strain, it was confirmed that there were removed parts of 36 base pairs (the part in bold in the sequence of SEQ ID NO: 4 below), when compared to the genome of the wild-type CD01-1821 strain on the genome. The amplification target sequence of the wild-type CD01-1821 strain and the amplification target sequence of the mutant CD01-1003 strain were shown below. The underlined parts are the primer sequences for amplifying the corresponding DNA fragment sequence, and the bold part indicates the sequence which is present in the wild-type CD01-1821 strain, but is removed in the mutant CD01-1003 strain.

[DNA fragment sequence for amplification of CD01-1821 strain (SEQ ID NO: 4)]

GCCAGGCAGCTGAATGTAATGGGATCACGGCAAGCTTCCAATACAGATTAG

ACCGCCCGGATCCCTTACAAAAGGCTGTGAGGCCAATGATCGATTGATCAATAGAT

AGTTAG**ATAGATAGATAGATAGATAGATAGATAGATAG**ATAGATAGATAG

ATAGATAGATAGATAGATAGATAGATAGATAGATAGATAGATAGATAGATAG

ATAGATAGATAGAAAACAAATACCTAGGCGATTACTGCTTCACTATAGCTTTT

TTCTCCTGCTTTTATCCGGCTGCCGTTTCGAGGCTTGGGCGAGGCGCCATTTCCCTTC

ACTCTTCTCCACAGCCA

[DNA fragment sequence for amplification of CD01-1003 strain (SEQ ID NO: 5)]

GCCAGGCAGCTGAATGTAATGGGATCACGGCAAGCTTCCAATACAGATTAG

ACCGCCCGGATCCCTTACAAAAGGCTGTGAGGCCAATGATCGATTGATCAATAGAT

AGTTAGATAGATAGATAGATAGATAGATAGATAGATAGATAGATAGATAGATAGAT

AGATAGATAGATAGATAGATAGATAGATAGAAAACAAATACCTAGGCGATTA

CTGCTTCACTATAGCTTTTTTCTCCTGCTTTTATCCGGCTGCCGTTTCGAGGCTTGGG

CGAGGCGCCATTTCCCTTCACTCTTCTCCACAGCCA

Primer A: 5'-GCCAGGCAGCTGAATGTAAT-3' (SEQ ID NO: 6) and primer B: 5'-TGGCTGTGGAGAAGAGTGAA-3' (SEQ ID NO: 7) amplifying these parts were selected, and using these, a PCR amplification reaction was performed. For the PCR reaction, after denaturation at 95°C for 5 minutes using a reaction solution containing taq polymerase, denaturation at 95°C for 10 seconds, annealing at 50°C for 10 seconds, and polymerization at 72°C for 15 seconds were repeated 35 times, and then a polymerization reaction was performed at 72°C for 5 minutes. The reaction solution amplified through the PCR process was under electrophoresis in 1.7% agarose gel and the size of the amplified DNA was confirmed (FIG. 1).

As a result, as shown in FIG. 1, it was confirmed that the amplified DNA fragments in the mutant CD01-1003 strain was in a size of about 313 bp, and had differences from the wild-type CD01-1821 strain in which DNA fragments in a size of about 349 bp were amplified.

Accordingly, through the result, it was confirmed that primer A: 5'-GCCAGGCAGCTGAATGTAAT-3'amd primer B: 5'-TGGCTGTGGAGAAGAGTGAA-3' could be used in order to select the mutant CD01-1003 strain.

From the above description, those skilled in the art to which the present application belongs will be able to understand that the present application can be implemented in other specific forms without changing technical spirit or essential features thereof. In this regard, examples described above should be understood in all respects as illustrative and not restrictive. The scope of the present application should be interpreted as all changed or modified forms derived from the meaning and scope of claims described below than the above detailed description and equivalent concepts thereof are included in the scope of the present application.

[ACCESSION NUMBER]

Name of Depository Authority: Korea Research Institute of Bioscience and Biotechnology Korea Collection for Type Culture (KCTC)
Accession number: KCTC14660BP
Date of deposit: 20210823
Name of Depository Authority: Korea Research Institute of Bioscience and Biotechnology Korea Collection for Type Culture (KCTC)
Accession number: KCTC15201BP
Date of deposit: 20221121

BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

# RECEIPT IN THE CASE OF AN ORIGINAL DEPOSIT

issued pursuant to Rule 7.1

TO: **CJ CheilJedang Corporation**

CJ CheilJedang Corporation

330, Dongho-ro, Jung-gu, Seoul

Republic of Korea

| I.  IDENTIFICATION OF THE MICROORGANISM | |
|---|---|
| Identification reference given by the DEPOSITOR:<br><br>*Schizochytrium* **sp. CD01-1821** | Accession number given by the INTERNATIONAL DEPOSITARY AUTHORITY:<br><br>**KCTC 14660BP** |

**II.  SCIENTIFIC DESCRIPTION AND/OR PROPOSED TAXONOMIC DESIGNATION**

The microorganism identified under I above was accompanied by:

   [   ] a scientific description

   [   ] a proposed taxonomic designation

(Mark with a cross where applicable)

**III.  RECEIPT AND ACCEPTANCE**

This International Depositary Authority accepts the microorganism identified under I above, which was received by it on **August 23, 2021.**

**IV.  RECEIPT OF REQUEST FOR CONVERSION**

The microorganism identified under I above was received by this International Depositary Authority on and a request to convert the original deposit to a deposit under the Budapest Treaty was received by it on

**V.  INTERNATIONAL DEPOSITARY AUTHORITY**

| | |
|---|---|
| Name: **Korean Collection for Type Cultures**<br><br>Address: Korea Research Institute of<br>     Bioscience and Biotechnology (KRIBB)<br>     181, Ipsin-gil, Jeongeup-si, Jeolllabuk-do 56212<br>     Republic of Korea | Signature(s) of person(s) having the power to represent the International Depositary Authority or of authorized official(s):<br><br>*Song-Gun Kim*<br><br>KIM, Song-Gun, Director<br>Date: **August 23, 2021** |

Form BP/4 (KCTC Form 17)                                                                                                    sole page

BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

# RECEIPT IN THE CASE OF AN ORIGINAL DEPOSIT

issued pursuant to Rule 7.1

TO: **CJ CHEILJEDANG CORPORATION**

CJ CHEILJEDANG CORPORATION

330 Dongho-ro, Jung-gu, Seoul

Republic of Korea

| I. IDENTIFICATION OF THE MICROORGANISM | |
|---|---|
| Identification reference given by the DEPOSITOR: <br><br> *Schizochytrium* **sp. CD01-1003** | Accession number given by the INTERNATIONAL DEPOSITARY AUTHORITY: <br><br> **KCTC 15201BP** |

II. SCIENTIFIC DESCRIPTION AND/OR PROPOSED TAXONOMIC DESIGNATION

The microorganism identified under I above was accompanied by:

   [   ] a scientific description

   [   ] a proposed taxonomic designation

(Mark with a cross where applicable)

III. RECEIPT AND ACCEPTANCE

This International Depositary Authority accepts the microorganism identified under I above, which was received by it on **November 21, 2022.**

IV. RECEIPT OF REQUEST FOR CONVERSION

The microorganism identified under I above was received by this International Depositary Authority on and a request to convert the original deposit to a deposit under the Budapest Treaty was received by it on

V. INTERNATIONAL DEPOSITARY AUTHORITY

| | Signature(s) of person(s) having the power to represent the International Depositary Authority or of authorized official(s): |
|---|---|
| Name: **Korean Collection for Type Cultures** <br><br> Address: Korea Research Institute of <br>     Bioscience and Biotechnology (KRIBB) <br>     181, Ipsin-gil, Jeongeup-si, Jeolllabuk-do 56212 <br>     Republic of Korea | *Song-Gun Kim* <br><br> KIM, Song-Gun, Director <br> Date: **November 21, 2022** |

Form BP/4 (KCTC Form 17)          sole page

**Claims**

1.   *Schizochytrium* sp. CD01-1003 strain that is a microalgae deposited under accession number KCTC15201BP, which produces bio-oil comprising a high concentration of DHA (Docosahexaenoic acid, 22:6).

2.   The strain according to claim 1, wherein the bio-oil comprises DHA of 25 to 35 % by weight based on the total weight of fatty acids.

3.   The strain according to claim 1, wherein the productivity of bio-oil of the strain is 44 to 54 g/L·day.

4.   The strain according to claim 1, wherein the productivity of DHA of the strain is 12.5 to 16.5 g/L·day.

5.   The strain according to claim 1, wherein the genomic DNA of the strain comprises a nucleotide sequence in which 36 nucleotides are deleted from the nucleotide sequence of SEQ ID NO: 4 of *Schizochytrium* sp. CD01-1821 strain, which is the parent strain.

6.   The strain according to claim 5, wherein the nucleotide sequence in which 36 nucleotides are deleted from the nucleotide sequence of SEQ ID NO: 4 consists of SEQ ID NO: 5.

7.   The strain according to claim 1, wherein the strain is selected with a primer set consisting of the nucleotide sequences of SEQ ID NO: 6 and SEQ ID NO: 7.

8.   A microbial product for producing DHA (Docosahexaenoic acid, 22:6) comprising the strain of any one claim of claim 1 to claim 7 or a culture solution thereof as an active ingredient.

9.   Biomass derived from a *Schizochytrium* sp. strain, comprising the strain of any one claim of claim 1 to claim 7, a culture solution of the strain, a dry matter of the culture solution, or a lysate of the dry matter.

10.   A feed composition, comprising the biomass derived from a *Schizochytrium* sp. strain of claim 9, or a concentrate, dry matter or extract of the biomass.

11.   A food composition, comprising the biomass derived from a *Schizochytrium* sp. strain of claim 9, or a concentrate, dry matter or extract of the biomass.

12.   A method for producing biomass derived from a *Schizochytrium* sp. strain, comprising; culturing the strain of any one claim of claim 1 to claim 7; and
recovering biomass containing DHA (Docosahexaenoic acid, 22:6) from the strain, a culture solution thereof, or a dry matter or lysate of the culture solution.

13.   A method for producing bio-oil derived from a *Schizochytrium* sp. strain, comprising; culturing the strain of any one claim of claim 1 to claim 7; and
recovering bio-oil containing DHA (Docosahexaenoic acid, 22:6) from the strain, a culture solution thereof, or a dry matter or lysate of the culture solution.

【FIG. 1】

## INTERNATIONAL SEARCH REPORT

| International application No. |
|---|
| **PCT/KR2023/015037** |

| A. | CLASSIFICATION OF SUBJECT MATTER |
|---|---|

**C12N 1/12**(2006.01)i; **C12P 7/6434**(2022.01)i; **A23K 10/16**(2016.01)i; **A23K 20/158**(2016.01)i; **A23L 29/00**(2016.01)i; **A23L 33/12**(2016.01)i; C12R 1/89(2006.01)n

According to International Patent Classification (IPC) or to both national classification and IPC

| B. | FIELDS SEARCHED |
|---|---|

Minimum documentation searched (classification system followed by classification symbols)

C12N 1/12(2006.01); A23K 10/16(2016.01); C12N 1/00(2006.01); C12P 23/00(2006.01); C12P 7/20(2006.01); C12P 7/64(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: DHA(docosahexaenoic acid), 바이오오일(biooil), 바이오매스(biomass), 스키조키트리움(Schizochytrium), CD01-1003, 미세조류(microalgae), 돌연변이(mutation)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | KR 10-2022-0080594 A (CJ CHEILJEDANG CORPORATION) 14 June 2022 (2022-06-14)<br>See abstract; claims 1-2, 7-9 and 14; paragraphs [0040] and [0080]-[0083]; and tables 1 and 3. | 1-13 |
| A | KR 10-1847551 B1 (INDUSTRIAL COOPERATION FOUNDATION JEONBUK NATIONAL UNIVERSITY et al.) 10 April 2018 (2018-04-10)<br>See entire document. | 1-13 |
| A | KR 10-2017-0032577 A (KOREA RESEARCH INSTITUTE OF BIOSCIENCE AND BIOTECHNOLOGY) 23 March 2017 (2017-03-23)<br>See entire document. | 1-13 |
| A | KR 10-2019-0110186 A (ADVANCED BIOMASS R&D CENTER) 30 September 2019 (2019-09-30)<br>See entire document. | 1-13 |

☑ Further documents are listed in the continuation of Box C.     ☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **11 January 2024** | **11 January 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
| --- |
| **PCT/KR2023/015037** |

**C.      DOCUMENTS CONSIDERED TO BE RELEVANT**

| Category* | Citation of document, with indication, where appropriate, of the relevant passages | Relevant to claim No. |
| --- | --- | --- |
| A | KR 10-2008-0111586 A (LEE, Joung Yeoul) 24 December 2008 (2008-12-24)<br>      See entire document. | 1-13 |

Form PCT/ISA/210 (second sheet) (July 2022)

## INTERNATIONAL SEARCH REPORT

| International application No. |
|---|
| **PCT/KR2023/015037** |

---

**Box No. I    Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

---

Form PCT/ISA/210 (continuation of first sheet) (July 2022)

**INTERNATIONAL SEARCH REPORT**
Information on patent family members

International application No.

**PCT/KR2023/015037**

| Patent document cited in search report | | | Publication date (day/month/year) | Patent family member(s) | | | Publication date (day/month/year) |
|---|---|---|---|---|---|---|---|
| KR | 10-2022-0080594 | A | 14 June 2022 | AU | 2021-398406 | A1 | 22 June 2023 |
| | | | | CA | 3198557 | A1 | 16 June 2022 |
| | | | | EP | 4257671 | A1 | 11 October 2023 |
| | | | | KR | 10-2550213 | B1 | 30 June 2023 |
| | | | | WO | 2022-124590 | A1 | 16 June 2022 |
| KR | 10-1847551 | B1 | 10 April 2018 | None | | | |
| KR | 10-2017-0032577 | A | 23 March 2017 | KR | 10-1777217 | B1 | 11 September 2017 |
| KR | 10-2019-0110186 | A | 30 September 2019 | None | | | |
| KR | 10-2008-0111586 | A | 24 December 2008 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020220175630

- US 5130242 A

**Non-patent literature cited in the description**

- **MIAO, X** ; **WU, Q**. *Biosource Technology*, 2006, vol. 97, 841-846